# EUROPEAN PATENT APPLICATION

(11) **EP 2 989 993 A1**
(43) Date of publication of application: **02.03.2016**
(21) Application number: 14787434.1
(22) Date of filing: 25.04.2014
(51) Int. Cl.: A61B 10/02, A61M 39/22

(54) **BIOPSY NEEDLE**

(30) Priority: 26.04.2013 KR 20130046849; 26.04.2013 KR 20130046859
(71) Applicant: Seoul National University Hospital, Seoul 110-744 (KR)
(72) Inventor: PARK, Chang Min, Seoul 110-744 (KR); GOO, Jin Mo, Seoul 110-744 (KR); LEE, Sang Min, Seoul 110-744 (KR)
(74) Representative: Stolmár & Partner Patentanwälte PartG mbB
(86) International application number: PCT/KR2014/003654
(87) International publication number: WO 2014/175698

(57) **Abstract**

Disclosed are a coaxial needle for guiding a biopsy needle during biopsy, the coaxial needle including a sheath provided with a passage for the biopsy needle, and a valve provided on the passage, or the coaxial needle including a sheath, an inner sheath passing through the inside of the sheath, and an inner stylet passing through the inside of the inner sheath; and a core biopsy needle to be penetrated into a body for collecting a tissue, the core biopsy needle including a sheath, and an inner stylet passing through the inside of the sheath, wherein a space for storing a cut-off tissue is provided at a distal end of the inner stylet.

## Description

### [Technical Field]

The present disclosure relates generally to a needle-type mechanism for use in the tissue biopsy, and more particularly, to distal end structures of a coaxial needle and a core biopsy needle for use in the tissue biopsy.

### [Background Art]

This section provides background information related to the present disclosure which is not necessarily prior art.

Typically, when a nodule or mass found in a human body is suspected of being cancer, tissue samples are collected and the pathological test is carried out on the collected tissue samples (which are called a tissue biopsy) to make a definite diagnosis. Overall, the tissue biopsy falls into two categories: methods of collecting tissues through a laparotomy and minimally invasive methods performed with the latest image-guided equipment. The former, surgical biopsy requires a larger incision such that a patient's recovery period and length of hospital stay are prolonged, the intensity of pain and complications gets higher and further the level of risk of death is increased. Thus, the biopsy based on the minimally invasive method has been a general trend in recent years. The image-guided biopsy can further be divided into an aspiration biopsy which only allows to collect cells, and a core biopsy which makes it possible to obtain more tissues.

A number of patents including U.S. Patent Nos. 3, 938, 505; 4, 314, 535; 5, 199, 441; 5, 469, 860; and 6,872,185 describe the aspiration biopsy. Also, a number of patents including U.S. Patent Nos. 3,477,423; 4,600,014; 5,161,542; 5,236,334; and 7,914,463 describe the core biopsy. The aspiration biopsy is mainly used in the cases where a pathologist is present in the biopsy site, or the possibility of cancer is so high that no immunological test or genetic analysis is not necessary, or an extensive necrosis is found in the tumor, or the risk of complications is very high. The core biopsy is mainly used in the cases where a more accurate diagnosis is required, or the genetic information on a cancer is obtained, or a patient is very likely to have a benign tumor, or the pathologist finds it difficult to give a diagnosis on the site, or the aspiration biopsy failed to give a diagnosis result. Biopsy needles for the biopsy are either core biopsy needles or aspiration biopsy needles, depending on the biopsy method applied.

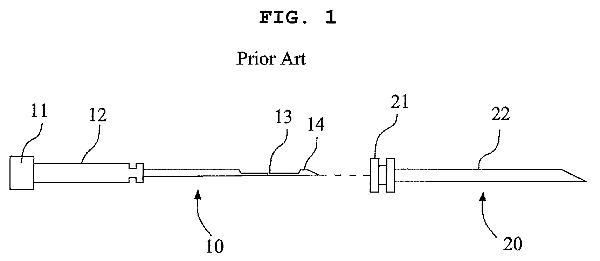

FIG. 1 shows an example of the structure of a core biopsy needle. This embodiment of the structure of a core biopsy needle illustrated in FIG. 1 is available from U.S. Patent No. 5,236,334, with some changes in terms and reference numerals, for convenience. The core biopsy needle has an inner stylet 10 and a sheath 20. The inner stylet 10 is composed of a hub 11, a body 12, a notch 13, and a distal end 14. The notch 13 is located between the distal end 14 and the body 12 and serves as a space for storing cut-off tissues. The distal end 14 is pointed at the end to be able to penetrate the body. The sheath 20 is composed of a hub 21 and a cannular 22.

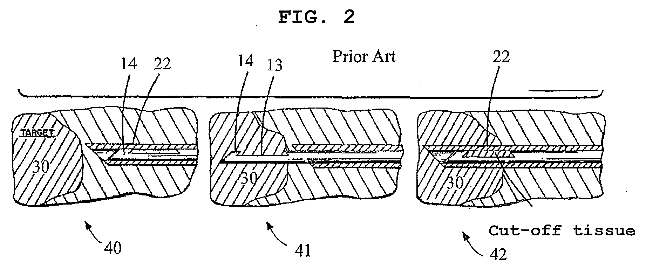

FIG. 2 shows an example of the method of using a core biopsy needle. This embodiment of the method of using a core biopsy needle is available from U.S. Patent No. 5,161,542, with some changes in terms and reference numerals, for convenience. First of all, an inner stylet and a sheath are inserted near a target tissue 30 to be collected (see 40). Then, a notch 13 is inserted into the tissue 30, with the help of a distal end 14 of the inner stylet (see 41). Following that, a cannular 22 is inserted into the tissue 30 in order to cut off the tissue that has entered into the notch 13 (see 42). Although not shown in the drawing, the core biopsy needle with the cut-off tissue still being kept inside the notch 13 is taken out of the body, and a test is performed on the tissue contained in the notch 13.

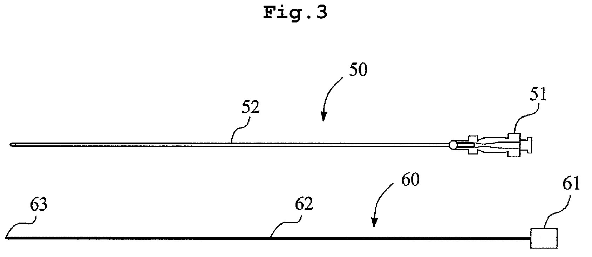

FIG. 3 shows an example of the structure of an aspiration biopsy needle. In general, this embodiment of the aspiration biopsy needle in FIG. 3 is broadly used and often known as a Chiba needle. The Chiba needle has a sheath 50 and an inner stylet 60. The sheath 50 is composed of a hub 51 and a cannular 52, and the inner stylet 60 is composed of a hub 61, a body 62 and a distal end 63. The inner stylet of the aspiration biopsy needle differs considerably from the inner style 10 of the core biopsy needle in that the former does not have a notch 13 serving as a space for storing a tissue collected.

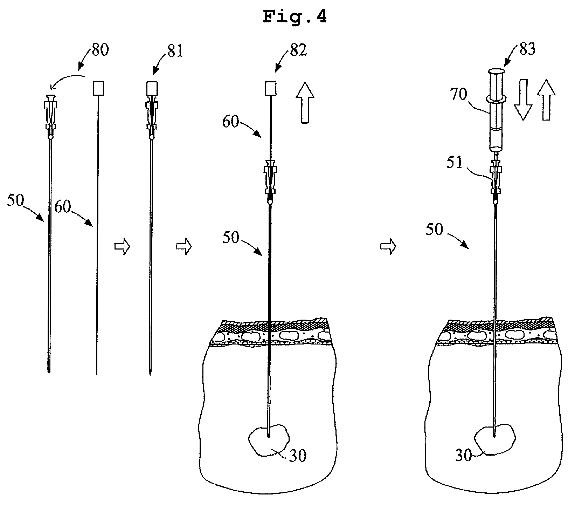

FIG. 4 shows an example of the method of using an aspiration biopsy needle. First of all, an inner stylet 60 and a sheath 50 are combined together (see 81) and then inserted up to a target tissue 30 to be collected (see 82). The inner stylet 60 is then separated from the sheath 50 (see 82). Next, a syringe 70 is connected to a hub 51 of the sheath 50 (see 83). Finally, a portion of the tissue is taken, applying the suction force of the syringe 70. Besides the syringe 70, different methods, such as a vacuum machine, may be employed as one of methods using the suction force. To improve the suction force, it is more desirable for the sheath 50 to have a smaller inner diameter. Thus, the outer diameter of the sheath 50 of an aspiration biopsy is normally smaller than the outer diameter of the sheath 20 of a core biopsy needle.

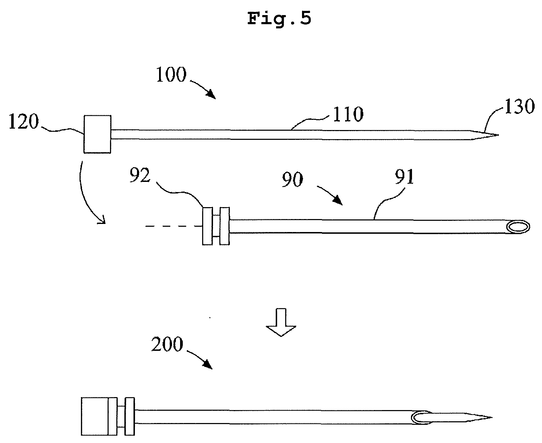

FIG. 5 shows a coaxial needle that can be used for the core biopsy. The coaxial needle has a sheath 90 and an inner stylet 100. The sheath 90 is composed of a cannular 91 and a hub 92, and the inner stylet 100 is composed of a body 110, a hub 120 and a distal end 130. The distal end 130 is pointed at the end to be able to penetrate the tissue. The sheath 90 and the inner stylet 100 of the coaxial needle are integrated as a combined form 200 before being inserted into the body. Once they are close to a target tissue to be collected, the inner stylet 100 is pulled out, and a core biopsy needle is inserted into the place where the inner stylet 100 was before. For a clearer manifestation, in FIG. 5, the inner stylet 100 in the combined form 200 is protruded considerably from the sheath 90. However, it is more desirable that the inner stylet is protruded less. When the core biopsy needs to be done several times on the same tissue, the coaxial needle is inserted into the body, the inner stylet 100 is then pulled out, and the sheath 90 is used as a passage for inserting the core biopsy needle into the body. In this manner, one may not insert the core biopsy needle every time into the body of a patient. In addition, the coaxial needle may be used as a passage for inserting the aspiration biopsy needle into the body.

While the coaxial needle has those advantages described above, there is a risk that air can enter the body when the coaxial needle is in use. That is, air can invade the body through the sheath 90, after the inner stylet 100 of the coaxial needle is pulled out before the core biopsy needle is inserted, such that air embolism may occur due to the air entered into the arteries of vital organs including a heart, the great vessels and a brain for example. Conventionally, in order to prevent this, an operator or user used to cover the entry of the hub 92 of the sheath 90 with his or her hand while using a coaxial needle, this only added the inconvenience to the operator, and such a manual process has not perfectly resolved the risk of having air entered the body, either.

Additionally, as the core biopsy and the aspiration biopsy have their own features as described above, they are usually employed individually for biopsy. Nonetheless, there are some occasions where the core biopsy and the aspiration biopsy are required at the same time for the same tissue. In these occasions, the core biopsy and the aspiration biopsy were performed separately and independently, or the core biopsy using a coaxial needle was first performed and then an aspiration biopsy needle was inserted via a passage created by the coaxial needle. In the former case, however, since needle insertion had to done for the core biopsy and the aspiration biopsy, respectively, a patient as well as an operator had to put up with inconveniences. Meanwhile, in the latter case, with the outer diameter of the sheath 50 of an aspiration biopsy needle being smaller than the outer diameter of the sheath 20 of a core biopsy needle, a gap was created between the inner surface of the cannular 91 of the coaxial needle and the outer surface of the cannular 52 of the aspiration biopsy needle, thereby raising the risk of air inflow through the gap.

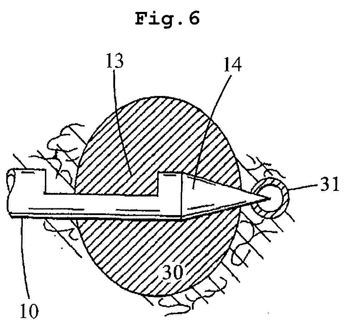

FIG. 6 shows a possible problem that might arise in the case of performing a core biopsy. This illustration in FIG. 6 is available from U.S. Patent No. 4, 708, 147, with some changes in terms and reference numerals, for convenience. In the case of a core biopsy, a gap exists between a distal end 14 provided to an inner stylet 10 and a notch 13 for storing a cut-off tissue, such that the distal end 14 often comes out of the tissue 30 to allow the cut-off tissue to be retained in the notch 13. When this occurs, the distal end 14 is likely to damage a blood vessel 31 that passes outside the tissue 30, or any organ. In particular, this has become more risky with the tendency of smaller sized tissues to be collected. Therefore, to address the issues mentioned above, there exists a need to improve the shape of the distal end of the inner stylet in a core biopsy needle.

### [Disclosure]

### [Technical Problem]

The problems to be solved by the present disclosure will be described in the latter part of the best mode for carrying out the invention.

### [Technical Solution]

This section provides a general summary of the disclosure and is not a comprehensive disclosure of its full scope or all of its features.

According to an aspect of the present disclosure, there is provided a coaxial needle for guiding a biopsy needle during biopsy, the coaxial needle including a sheath provided with a passage for the biopsy needle; and a valve provided on the passage.

According to another aspect of the present disclosure, there is provided a coaxial needle including a sheath, an inner sheath, and an inner stylet.

According to still another aspect of the present disclosure, there is provided a core biopsy needle to be penetrated into a body for collecting a tissue, the core biopsy needle including a sheath, and an inner stylet, wherein a space for storing the tissue is provided at a distal end of the inner stylet.

### [Advantageous Effects]

The advantageous effects of the present disclosure will be described in the latter part of the best mode for carrying out the invention.

### [Description of Drawings]

FIG. 1 is a view showing an example of the structure of a core biopsy needle.
FIG. 2 is a view showing an example of the method of using a core biopsy needle.
Fig. 3 is a view showing an example of the structure of an aspiration biopsy needle.
FIG. 4 is a view showing an example of the method of using an aspiration biopsy needle.
FIG. 5 is a view showing an example of the structure of a coaxial needle.
FIG. 6 is a view illustrating a possible problem that may arise in the case of core biopsy.
FIG. 7 is a front view of an example of the sheath of a coaxial needle according to the present disclosure (Embodiment 1).
FIG. 8 is a top view of said example of the sheath of a coaxial needle according to the present disclosure (Embodiment 1).
FIG. 9 is a view showing said example of the sheath of a coaxial needle according to the present disclosure (Embodiment 1) when in use.
FIG. 10 is a front view of another example of the sheath of a coaxial needle according to the present disclosure (Embodiment 2).
FIG. 11 is a top view of said another example of the sheath of a coaxial needle according to the present disclosure (Embodiment 2).
FIG. 12 is a view showing said another example of the sheath of a coaxial needle according to the present disclosure (Embodiment 2) when in use.
FIG. 13 is a front view of a still another example of the sheath of a coaxial needle according to the present disclosure (Embodiment 3).
FIG. 14 is a top view of said still another example of the sheath of a coaxial needle according to the present disclosure (Embodiment 3).
FIG. 15 is a view showing said still another example of the sheath of a coaxial needle according to the present disclosure (Embodiment 3) when in use.
FIG. 16 is a view showing that an opening through which the biopsy needle in Embodiment 2 and Embodiment 3 can pass is blocked by an air barrier.
FIG. 17 is a front view of a yet another example of the sheath of a coaxial needle according to the present disclosure (Embodiment 4).
FIG. 18 is a top view of said yet another example of the sheath of a coaxial needle according to the present disclosure (Embodiment 4).
FIG. 19 is a view showing an example of a coaxial needle according to the present disclosure.
FIG. 20 is a view showing an example of the usage of the coaxial needle according to the present disclosure.
FIG. 21 is a side view of an example of a core biopsy needle according to the present disclosure.
FIG. 22 is a view showing the core biopsy needle according to the present disclosure, which is arranged at an angle.
FIG. 23 is a top view showing an example of the core biopsy needle according to the present disclosure.
FIG. 24 is a view showing an example of using the core biopsy needle according to the present disclosure.
FIG. 25 is a view showing a tissue storage space with reinforcements on the sides, in the core biopsy needle according to the present disclosure.

### [Best Mode for Carrying Out Invention]

Hereinafter, the present disclosure will now be described in detail with reference to the accompanying drawings.

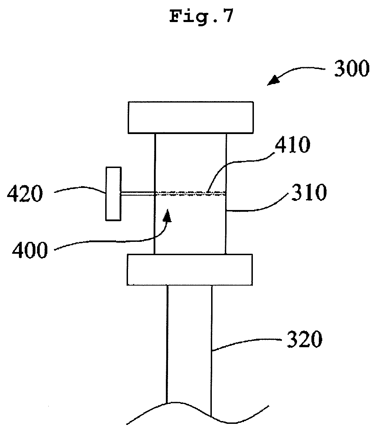

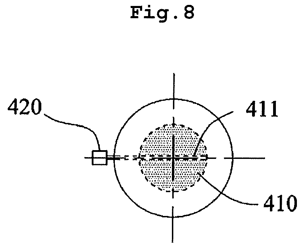

FIGS. 7 and 8 show an example of a coaxial needle according to the present disclosure (Embodiment 1). FIG. 7 is a front view, and FIG. 8 is a top view taken from above. The coaxial needle according to the present disclosure has a sheath 300 composed of a hub 310 and a cannular 320. In the drawings, the cannular 320 is shown only partially. A valve 400 disposed in the hub 310 includes a plate 410 and a handle 420. The handle 420 is provided to help an operator to be able to rotate the plate 410 and to able to check an open or closed state of the valve 400 in an easier manner.

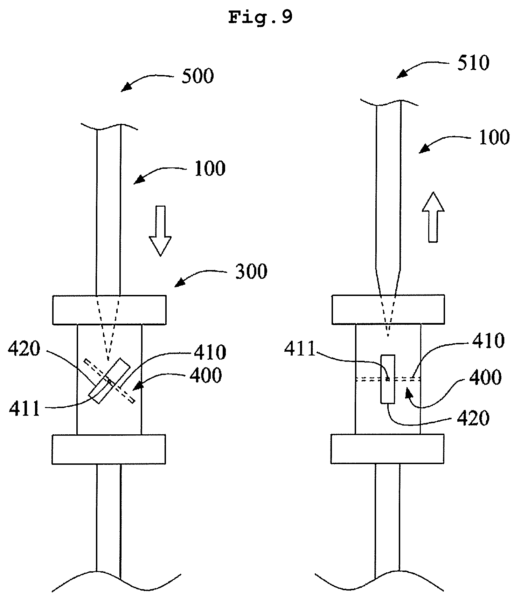

FIG. 9 shows said example of the coaxial needle according to the present disclosure (Embodiment 1) when in use. When the inner stylet 100 of the coaxial needle is inserted into the sheath 300 of the coaxial needle, the valve 400 has to be in an open state; when the inner stylet 100 is pulled out, the valve has to be in a closed state. This is accomplished by rotating the plate 410 during the insertion of the inner stylet 100 using the handle connected to the rotational axis 411 of the plate 410, thereby causing the valve 400 to be open (see 500), and turning the handle 420 once the inner stylet 100 of the coaxial needle is pulled out to cause the valve 400 to be closed (see 510). Based on the relative locational relationship between the handle 420 and the plate 410, one can easily find out from outside whether the valve 400 is open or closed. In other words, the plate 410 and the handle 420 are arranged such that the plane of the plate and the longitudinal direction of the handle are at right angles to each other. Therefore, when the handle 420 is positioned upright or vertically, it indicates that the valve 400 is closed (see 510); when the handle 420 is slanted, it indicates that the valve 400 is open (see 500). This locational relationship between the plate 410 and the handle 420 also works where they are disposed in the other way around. It is sufficient to understand that the open or closed state of the valve 400 can easily be checked from outside based on the relative locational relationship between the handle 420 and the plate 410.

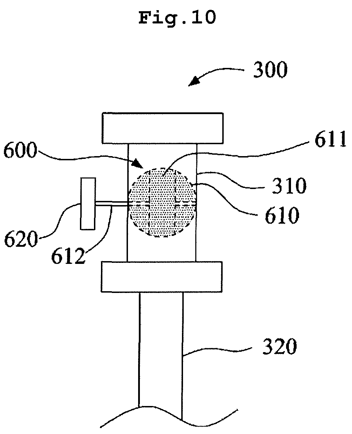

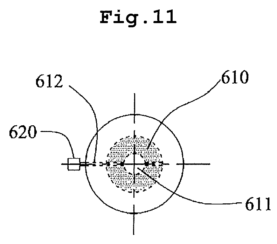

FIGS. 10 and 11 show another example of the sheath 300 of the coaxial needle according to the present disclosure (Embodiment 2). FIG. 10 is a front view, and FIG. 11 is a top view taken from above. A valve 600 disposed in a hub 310 includes a sphere 610 having an opening 611 for allowing a biopsy needle to pass therethrough, and a handle 620 connected to the rotational axis of the sphere 610. The sphere 610 is configured to be rotatable in order to open or close the valve 600. The handle 620 is configured to be turned directly by an operator, or designed such that the operator can easily check the open or closed state of the valve. The sphere 610 is preferably made of flexible materials like rubber, as it is intended to make tight contact with the inner side of the hub 310.

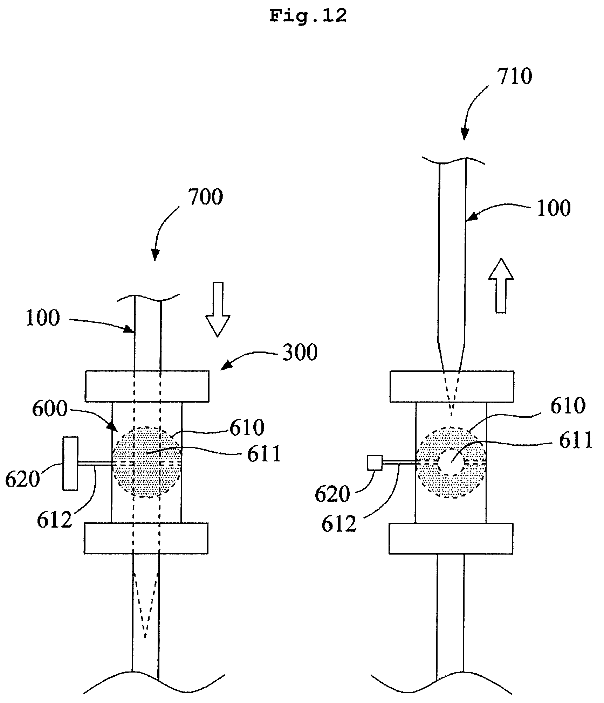

FIG. 12 shows said another example of the sheath of a coaxial needle 300 according to the present disclosure (Embodiment 2) when in use. In this embodiment, when a handle 620 connected to the rotational axis of a sphere 610 is turned and an opening in the sphere through which a biopsy needles passes is positioned vertically, the valve 600 is open (see 700); when the opening 611 is positioned horizontally, the valve 600 is closed (see 710). Again, the relative locational relationship between the handle 620 and the opening 611 through which the biopsy needle can pass helps an operator to easily check the open or closed state of the valve 600 from outside. By placing the longitudinal direction of the opening 611 through which the biopsy needle can pass and the longitudinal direction of the handle 620 in parallel, the position of the handle 620 can be indicative of whether the opening 611 is positioned upright or tilted horizontally (see 700 and 710). The locational relationship of the handle 620 also works in the other way around. By way of example, FIG. 11 illustrated the case where the inner stylet 100 is either inserted into or pulled out of the sheath 300.

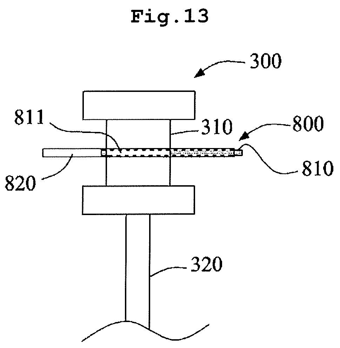

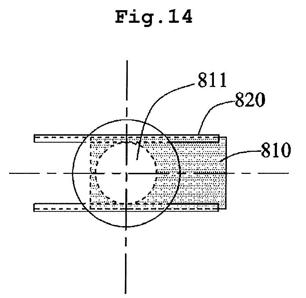

FIGS. 13 and 14 show a still another example of the sheath 300 of the coaxial needle according to the present disclosure (Embodiment 3). FIG. 13 is a front view, and FIG. 14 is a top view taken from above. A valve 800 disposed in a hub 310 includes a plate 810 having an opening 811 for allowing a biopsy needle to pass therethrough, and a guide 820 along which the plate can move. The plate 810 is configured to be movable by the guide 820 to enable opening or closing the valve 800.

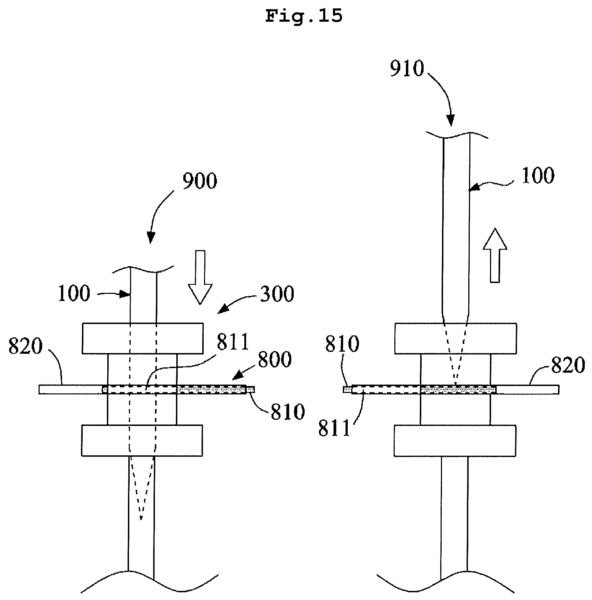

FIG. 15 shows said still another example of the sheath of a coaxial needle according to the present disclosure (Embodiment 3) when in use. When the opening 811 portion of the plate 810 is pushed into the sheath 300 by the guide 820, the valve 800 is open (see 900); when a portion without an opening is pushed into the sheath 300 by the guide 820, the valve 800 is closed (see 910). By way of example, FIG. 15 illustrated the case where the inner stylet 100 is either inserted into or pulled out of the sheath 300.

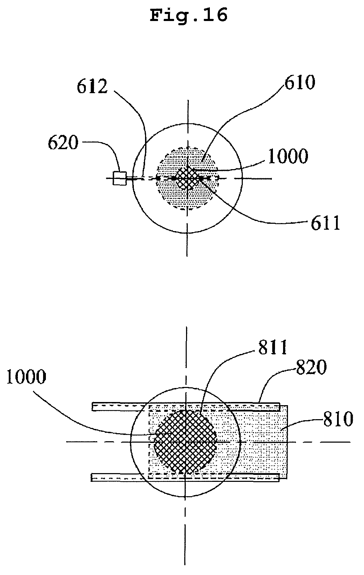

FIG. 16 shows that the openings 611, 811 through which the biopsy needle in the Embodiments 2 and 3 can pass are blocked by air barriers 1000. That is, the top views in FIGS. 11 and 14 corresponding to Embodiments 2 and 3, respectively, are now provided with the air barriers 1000, as illustrated in FIG. 16. This air barrier is made of materials that are permeable to air but impermeable to a biopsy needle. A preferable example of the materials is rubber. In this way, air cannot be invaded into the valve 600, 800 even when the valve 600, 800 is open. Better yet, the valve 600, 800 should be maintained in the closed state for complete blockage of air.

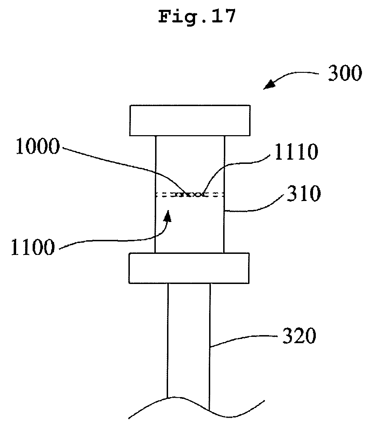

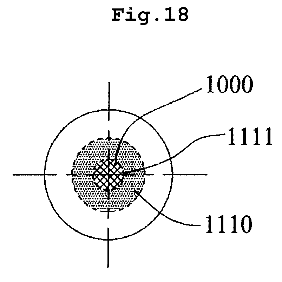

FIGS. 17 and 18 each show a yet another example of the sheath 300 of the coaxial needle according to the present disclosure (Embodiment 4).

FIG. 17 is a front view, and FIG. 18 is a top view taken from above. A valve disposed in a hub 310 includes a plate 1110 having an opening 1111 for allowing a biopsy needle to pass therethrough, and an air barrier 1000. Although now shown, the valve in Embodiment 4 may be in a sphere form with an opening for allowing a biopsy needle to pass therethrough as in Embodiment 2. Unlike those valves in Embodiments 1 and 3 where the valves are either open or closed, the valve in Embodiment 4 serves to pass through the biopsy needle but to block air by using the air barrier, with the valve being kept in the closed state at all times.

While the valves 400, 600, 800, 1100 in Embodiments 1, 2, 3 and 4 are envisaged to be in the hub 310, their locations can be anywhere on the passage of the sheath 300, along which the biopsy needle goes by. Therefore, the valves may be in the cannular 320 of the sheath 300. In addition, for efficient blockage of air, Embodiment 1 to Embodiment 4 can be combined as well.

The present disclosure features an openable/closable valve provided on the passage of the sheath of the coaxial needle. However, a person skilled in the art will understand that any variations, alterations or modifications of such an openable/closable valve fall within the scope of the present disclosure and are not excluded.

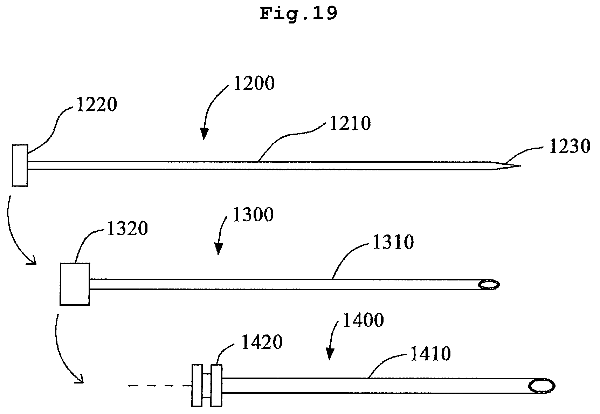

FIG. 19 shows an example of the coaxial needle according to the present disclosure. The coaxial needle in this disclosure is composed of an inner sheath 1300, a sheath 1400, and an inner stylet 1200. The sheath 1400 is located on the outermost part, followed by the inner sheath 1300 passing through the sheath 1400 and then the inner stylet 1200 passing through the inner sheath 1300 in an inward direction. Each of the inner sheath 1300 and the sheath 1400 is composed of a cannular 1310, 1410 and a hub 1320, 1420. The inner stylet 1200 is composed of a body 1210, a hub 1220, and a distal end 1230. The distal end 1230 is pointed at the end to be able to penetrate the body. It is desirable that the dimensions of an inner diameter of the cannular 1310 of the inner sheath 1300 should be determined by the dimensions of an outer diameter of an aspiration biopsy needle, as the space defined by the inner diameter of the cannular 1310 is used for a passage for the aspiration biopsy needle to get into the body. To this end, the outer surface of the cannular 52 of the aspiration biopsy needle preferably comes into contact with the inner surface of the cannular 1310 of the inner sheath 1300. This can prevent air from entering the body when the aspiration biopsy needle is inserted into the inner sheath 1300. Similarly, it is desirable that the dimensions of an inner diameter of the cannular 1410 of the sheath 1400 should be determined by the dimensions of an outer diameter of a core biopsy needle, as the space defined by the inner diameter of the cannular 1410 is used for a passage for the core biopsy needle to get into the body. Because it is the distal end 1230 of the inner stylet 1200 that is penetrated into the body, the end portions of the bodies 1310, 1410 opposite to the hubs 1320, 1420 of the sheath 1400 and inner sheath 1300 do not have to be in a pointed form. Nonetheless, it is more desirable that they are pointed at the end, in order to facilitate the penetration into the body.

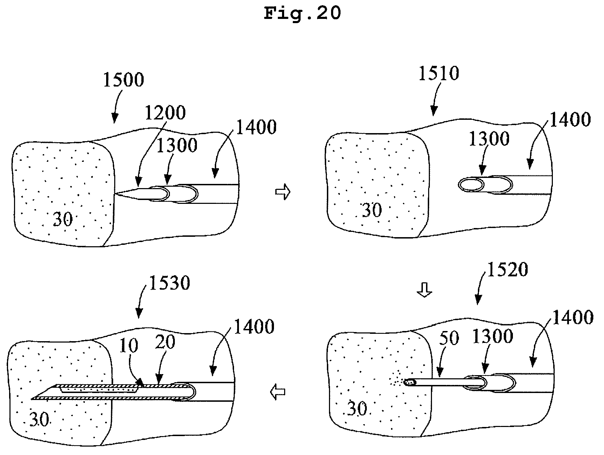

FIG. 20 shows the process of a core biopsy and an aspiration biopsy on the same tissue, according to the present disclosure. First of all, a coaxial needle is inserted close to a target tissue 30 to be collected (see 1500). Next, the inner stylet 1200 of the coaxial needle is pulled out (see 1510). Following that, an aspiration biopsy needle is pushed into the place where the inner stylet 1200 was before (see 1520). In FIG. 20, the sheath 50 of the aspiration biopsy needle in FIG. 3 is illustrated. The aspiration biopsy method using such an aspiration biopsy needle is same as the method described in FIG. 4. After pulling out the aspiration biopsy needle and the inner sheath 1300, a core biopsy needle is inserted into the place where the inner sheath 1300 was before (see 1530). In FIG. 20, the sheath 20 and the inner stylet 10 of the core biopsy needle in FIG. 1 are illustrated. The subsequent core biopsy method is same as the method described in FIG. 2.

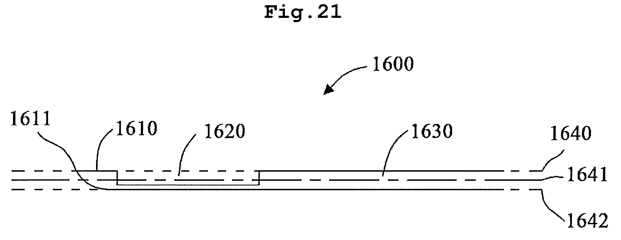

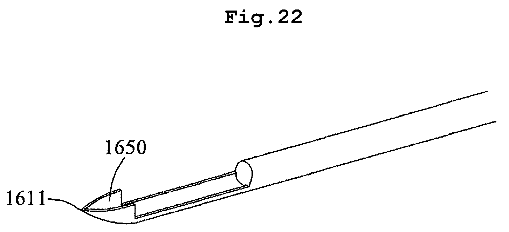

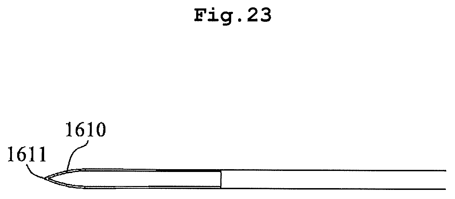

FIGS. 21, 22 and 23 show a side view, a top view and a view at an angle, respectively, of the inner stylet used in the core biopsy needle according to the present disclosure. The inner stylet 1600 according to the present disclosure includes a distal end 1610, a notch 1620, and a body 1630 as in the conventional ones, except that the distal end 1610 has a space 1650 for storing a cut-off tissue therein. To prevent the cut-off tissue kept in the notch 1620 or in the tissue storage space 1650 of the distal end 1610 from getting back into the body, the inner stylet, as seen from the side (FIG. 21), is preferably arranged such that an end point 1611 of the distal end 1610 lies above a center line 1641. Here, the center line 1641 refers to a line that crosses the center between an upper line 1640 running over the upper side of the body 1630 and a lower line 1642 running over the lower side of the body. More preferably, the end point 1611 of the distal end 1610 coincides with the upper line 1640 so that an open entry of the sheath when the inner stylet 1600 is pushed into the sheath is blocked. Moreover, to enable the distal end 1610 of the inner stylet 1600 to have better resistance against a target tissue and easily penetrate the tissue, the distal end 1610 is preferably in a streamlined form, when the inner stylet 1600 is seen from above (FIG. 23).

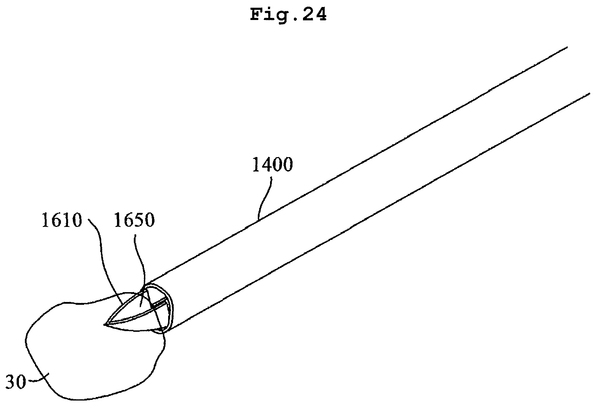

FIG. 24 shows an example of the usage of the core biopsy needle according to the present disclosure. If a target tissue 30 required for biopsy is small, only the distal end 1610 is inserted into the tissue, and the sheath 1400 is moved to cut off the tissue contained in the space 1650 in the distal end, thereby making it possible to ensure to obtain a tissue required for biopsy from the body.

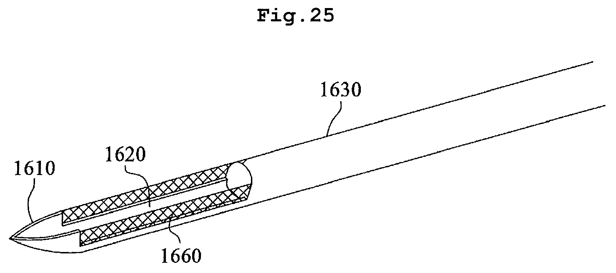

FIG. 25 shows a tissue storage space with reinforcements on the sides, in the core biopsy needle according to the present disclosure. In the case of FIG. 21, lateral sides of the notch were open to allow more tissues could fit into the notch 1620. However, in that case, a joining part between the distal end 1610 and the body 1630 did not get a strong support such that if a target tissue was hard, the joining part could bend and the distal end 1610 could be deviated from its travel direction. To resolve this problem and to enable easier entry of the tissue into the notch 1620, porous side walls 1660, i.e. having openings on the lateral faces, may be provided. Then the tissue can enter through these openings in the side walls 1660.

It will now be described with respect to various embodiments of the present disclosure.
(1) A coaxial needle for guiding a biopsy needle during biopsy, the coaxial needle characterized by comprising:
   a sheath provided with a passage for the biopsy needle; and
   a valve provided on the passage.
(2). The coaxial needle according to (1), characterized in that the valve is in a sphere form with an opening for allowing the biopsy needle to pass therethrough.
(3).The coaxial needle according to (2), characterized in that the sphere is made of rubber materials.
(4).The coaxial needle according to (2), characterized in that the sphere rotates around a rotational axis.
(5).The coaxial needle according to (2), characterized in that the opening in the sphere for allowing the biopsy needle to pass therethrough is blocked with an air barrier.
(6). The coaxial needle according to (4), characterized by comprising a handle connected to the rotational axis of the sphere.
(7).The coaxial needle according to (6), characterized in that longitudinal direction of the handle connected to the rotational axis of the sphere is in parallel to longitudinal direction of the opening in the sphere for allowing the biopsy needle to pass therethrough.
(8).The coaxial needle according to (1), characterized in that the valve is in a plate form having an opening for allowing the biopsy needle to pass therethrough.
(9).The coaxial needle according to (8), characterized in that the plate has a guide that can slidably get into the passage provided in the sheath where the biopsy needle goes by.
(10). The coaxial needle according to (8), characterized in that the opening in the plate for allowing the biopsy needle to pass therethrough is blocked with an air barrier.
(11). The coaxial needle according to (10), characterized in that the air barrier is made of rubber materials.
(12). The coaxial needle according to (1), characterized in that the valve is in a plate form.
(13). The coaxial needle according to (12), characterized in that the plate is rotatable.
(14). The coaxial needle according to (13), characterized by comprising a handle connected to a rotational axis of the plate.
(15). The coaxial needle according to (14), characterized in that longitudinal direction of the handle connected to the rotational axis of the plate is at right angles with a horizontal plane of the plate.
(16). A coaxial needle, characterized by comprising: a sheath; an inner sheath passing through the inside of the sheath; and an inner stylet passing through the inside of the inner sheath.
(17). The coaxial needle according to (16), characterized in that the inner stylet has a pointed distal end.
(18). The coaxial needle according to (16), characterized in that the inner sheath has a cannular with an inner surface coming into contact with an outer surface of a cannular of an aspiration biopsy needle.
(19). A method of using a coaxial needle having the structure described in any of (16) to (18), the method comprising pulling out an inner stylet and then inserting an aspiration biopsy needle into an inner sheath.
(20). A core biopsy needle to be penetrated into a body for collecting a tissue, characterized by comprising: a sheath; and an inner stylet passing through the inside of the sheath, wherein a space for storing a cut-off tissue is provided at a distal end of the inner stylet.
(21). The core biopsy needle according to (20), characterized in that an end point of the distal end is located between a center line and an upper line, as seen from the inner stylet.
(22). The core biopsy needle according to (20), characterized in that an end point of the distal end coincides with an upper line, as seen from the inner stylet.
(23). The core biopsy needle according to (20), characterized in that the distal end has a streamlined form, as the inner stylet is seen from above.
(24). The core biopsy needle according to (20), characterized by comprising side walls encompassing a notch of the inner stylet.
(25). The core biopsy needle according to (24), characterized in that the side walls have openings for allowing the entry of tissues.

To summarize, with the coaxial needle according to the present disclosure, it becomes easier to block air from entering the body through the sheath of the coaxial needle in those cases where a core biopsy needle is to be inserted into the place where the inner stylet of the coaxial needle was before it was pulled out, or the core biopsy needle is replaced several times, thereby increasing user convenience and effectively avoiding any risk factors that occur in a patient.

With the coaxial needle according to the present disclosure, an operator or user can easily check the open or closed state of the valve.

With the coaxial needle according to the present disclosure, the aspiration biopsy and the core biopsy can be performed concurrently on the same tissue in a simpler manner.

With the core biopsy needle according to the present disclosure, the risk of damaging vessels or organs due to the displacement of the distal end during collecting a tissue can be lowered, as the distal end of the inner stylet also acts as a notch for storing the tissue.

## Claims

1. A coaxial needle for guiding a biopsy needle during biopsy, the coaxial needle **characterized by** comprising:
a sheath provided with a passage for the biopsy needle; and
a valve provided on the passage.

2. The coaxial needle according to claim 1, **characterized in that** the valve is in a sphere form with an opening for allowing the biopsy needle to pass therethrough.

3. The coaxial needle according to claim 2, **characterized in that** the sphere is made of rubber materials.

4. The coaxial needle according to claim 2, **characterized in that** the sphere rotates around a rotational axis.

5. The coaxial needle according to claim 2, **characterized in that** the opening in the sphere for allowing the biopsy needle to pass therethrough is blocked with an air barrier.

6. The coaxial needle according to claim 4, **characterized by** comprising a handle connected to the rotational axis of the sphere.

7. The coaxial needle according to claim 6, **characterized in that** longitudinal direction of the handle connected to the rotational axis of the sphere is in parallel to longitudinal direction of the opening in the sphere for allowing the biopsy needle to pass therethrough.

8. The coaxial needle according to claim 1, **characterized in that** the valve is in a plate form having an opening for allowing the biopsy needle to pass therethrough.

9. The coaxial needle according to claim 8, **characterized in that** the plate has a guide that can slidably get into the passage provided in the sheath where the biopsy needle goes by.

10. The coaxial needle according to claim 8, **characterized in that** the opening in the plate for allowing the biopsy needle to pass therethrough is blocked with an air barrier.

11. The coaxial needle according to claim 10, **characterized in that** the air barrier is made of rubber materials.

12. The coaxial needle according to claim 1, **characterized in that** the valve is in a plate form.

13. The coaxial needle according to claim 12, **characterized in that** the plate is rotatable.

14. The coaxial needle according to claim 13, **characterized by** comprising a handle connected to a rotational axis of the plate.

15. The coaxial needle according to claim 14, **characterized in that** longitudinal direction of the handle connected to the rotational axis of the plate is at right angles with a horizontal plane of the plate.

16. A coaxial needle, **characterized by** comprising:
a sheath;
an inner sheath passing through the inside of the sheath; and
an inner stylet passing through the inside of the inner sheath.

17. The coaxial needle according to claim 16, **characterized in that** the inner stylet has a pointed distal end.

18. The coaxial needle according to claim 16, **characterized in that** the inner sheath has a cannular with an inner surface coming into contact with an outer surface of a cannular of an aspiration biopsy needle.

19. A method of using a coaxial needle having the structure described in any of Claim 16 to Claim 18, the method comprising pulling out an inner stylet and then inserting an aspiration biopsy needle into an inner sheath.

20. A core biopsy needle to be penetrated into a body for collecting a tissue, **characterized by** comprising:
a sheath; and
an inner stylet passing through the inside of the sheath,
wherein a space for storing a cut-off tissue is provided at a distal end of the inner stylet.

21. The core biopsy needle according to claim 20, **characterized in that** an end point of the distal end is located between a center line and an upper line, as seen from the inner stylet.

22. The core biopsy needle according to claim 20, **characterized in that** an end point of the distal end coincides with an upper line, as seen from the inner stylet.

23. The core biopsy needle according to claim 20, **characterized in that** the distal end has a streamlined form, as the inner stylet is seen from above.

24. The core biopsy needle according to claim 20, **characterized by** comprising side walls encompassing a notch of the inner stylet.

25. The core biopsy needle according to claim 24, **characterized in that** the side walls have openings for allowing the entry of tissues.
